# EUROPEAN PATENT APPLICATION

(11) **EP 4 822 646 A1**
(43) Date of publication of application: **14.10.2026**
(21) Application number: 25169528.4
(22) Date of filing: 09.04.2025
(51) Int. Cl.: G06T 11/00, G06T 5/94, G06T 7/00, A61B 6/50

(54) **SYSTEM AND METHOD FOR GENERATING MR-VIRTUAL X-RAY IMAGES**

(71) Applicant: Siemens Healthineers AG, 91301 Forchheim (DE); Universität Zürich, 8006 Zürich (CH)
(72) Inventor: Sommer, Stefan, 8005 Zürich (CH); Schmaranzer, Florian, 8404 Winterthur (CH)
(74) Representative: Fischer, Michael

(57) **Abstract**

The invention relates to a system (100) and method (200) for generating an MR-based-virtual X-Ray, hereafter MRXR, image, said method (200) comprising:
- acquiring (201) an initial set of one or several MRI images, wherein the MRI images comprised within said initial set are configured for enabling one or several 3D representations of a biological object;
- processing (202) said one or several MRI images for increasing, notably for each of said one or several images, a visibility of each image region, if any, that represents a bone of the biological object - called hereafter "bone-region" - with respect to a visibility of another region that represents tissue of the biological object that is free of bone - called hereafter "non-bone region", and creating an image input set comprising the processed one or several images;
- feeding (203) an improved bone visibility - hereafter IBV - 3D image into a cone-beam projection algorithm configured for performing an X-Ray-like projection, wherein the IBV 3D image is obtained from the processed one or several images of said input set, and wherein the cone-beam projection algorithm is configured for outputting said MRXR image;
- providing (204) the outputted MRXR image via an interface.

## Description

### Technical Field

The present invention falls within the field of Magnetic Resonance Imaging (MRI) and proposes a system and a method for generating Magnetic Resonance (MR)-based virtual X-ray images.

### Background Art

Diagnostics and/or surgery planning often require patients to undergo different imaging modalities such as X-Ray, Computer Tomography (CT), and MR. For instance, radiographs and scans are the standard imaging modalities for diagnosing bone-related diseases, deformities, and are typically used for surgical planning. Unfortunately, contrary to MRI which offers detailed, radiation-free imaging of soft tissues, X-Ray imaging techniques however are based on ionizing radiation, raising concerns about cumulative exposure, especially in younger patients. In particular, despite the availability of 3D data from CT, some of the diagnostic criteria are still evaluated on conventional X-rays (e.g. the alpha-angle to evaluate femoroacetabular impingement deformity).

It is therefore desired, to find a solution for reducing exposure to X-ray radiations, notably in the context of diagnostics or surgery planning requiring different imaging modalities. Furthermore, it is also desired to reduce the number of imaging apparatuses required for performing said different imaging modalities.

### Summary of Invention

An objective of the present invention is to propose a method and a system capable of eliminating the exposure to X-Ray radiation and a number of imaging apparatuses that are conventionally used for performing different imaging modalities in order to increase the safety and comfort of a patient.

This objective is achieved by the measures taken in accordance with the independent claims. Further advantageous embodiments are proposed by the dependent claims.

The present invention concerns also a method, preferentially a computer-implemented method, for generating an MR-based virtual X-Ray image, hereafter MRXR image, the method comprising:
- acquiring an initial set of one or several MRI images, wherein the MRI images comprised within said initial set are configured for enabling one or several 3D representations of a biological object. According to the present invention, it is considered that said 3D representation comprises at least a part representing a bone of the biological object, called hereafter the bone part, and another part representing a biological object tissue softer than said bone, called hereafter the non-bone part;
- processing said one or several MRI images of the initial set for increasing, notably for each of said one or several images, a visibility of each image region, if any, that represents at least a part of a bone (notably cortical bone) of the biological object (i.e. that represents said bone part of the 3D representation) - also called hereafter the "bone region - with respect to a visibility of another region that represents tissue of the biological object that is free of a bone (i.e. that represents said non-bone part of the 3D representation) - also called hereafter the non-bone region, and creating an image input set comprising the processed one or several images;
- feeding an improved bone visibility - hereafter IBV - 3D image into a cone-beam projection algorithm configured for performing an X-Ray-like projection, wherein the IBV 3D image is obtained from the processed one or several images of said input set, and wherein the cone-beam projection algorithm is configured for outputting said MRXR image;
- providing the outputted MRXR image via an interface.

The present invention concerns also a system, preferentially a computer-implemented system, said system comprising:
- a first interface for acquiring an initial set of one or several MRI images, wherein the MRI images comprised within said initial set are configured for enabling one or several 3D representations of a biological object, wherein said 3D representation typically comprises a part representing a bone of the biological object, called hereafter the bone part, and another part representing a biological object tissue softer than said bone, called hereafter the non-bone part;
- a processing unit comprising a processor and a memory, said processing unit being configured for processing said one or several MRI images;
wherein said processing unit is further configured for carrying out the steps of the previously described method.

### Description of Embodiments

The present invention proposes to use one or several MRI images of a biological object to create the so-called MRXR image. For this purpose, bone visibility in said one or several MRI images is increased with respect to the visibility of other tissue(s) of the biological object.

Preferentially, the initial set comprises several two-dimensional (2D) MRI images that enable said 3D representation of the biological object, which can be typically reconstructed from the 2D MRI images of the initial set. Each of said 2D MRI image may represent a slice of the biological object, the image comprising voxels (aligned or spread in 2D) whose respective intensity is related to the type of tissue that is currently imaged. Preferentially, the method according to the invention may comprise normalizing a signal intensity (i.e. the voxel intensity) of the 2D MRI images of said initial set.

According to another preferred embodiment, the initial set may comprise one (a single) MRI image that is a 3D MRI image (i.e. voxels aligned or spread in the three dimensions) that represents the biological object. Said initial set of 2D images or 3D MRI image might be acquired by the system according to the invention, for instance directly from an MRI apparatus to which it might be connected for controlling the acquisition of said one or several images, or might be received by the system as an input provided for instance by an operator. Optionally, the initial set may comprise several 3D MRI images, representing for instance each a different MRI contrast. Said 3D MRI images of the initial set might then be processed for increasing a bone visibility in each of them, and then used for constructing or creating said IBV 3D image, for instance by merging them into a single 3D image that is said IBV 3D image.

In order to make the one or several images of the initial set in a "format" or "configuration" that is appropriate for creating the MRXR image, said one or several MRI images need to be processed for rendering any bone region appearing in said image(s) more visible (e.g. brighter bone region, or alternatively darker non-bone (that represents notably tissue softer than bone, notably cortical bone) and background region(s) if considering an initial set comprising intensity inverted MRI images. For instance, said processing said one or several images may comprise, determining, for each of the images of the initial set, if said image comprises a region that represents said bone of the biological object, and, in the affirmative, replacing said image by a contrast-altered image (that is the processed image in this case), wherein the contrast-altered image is obtained by altering a contrast of the considered image for increasing said visibility of said bone region, or at least a part of said bone region, with respect to the visibility of said non-bone region (e.g. by rendering at least said part of said bone region brighter than said another region), otherwise (i.e. if the image does not comprise any region representing the bone-part), preferentially, keeping the image unchanged. In particular, the system according to the invention might be configured for implementing an automatic identification, within the initial set of one or several MRI images, of MRI images that comprise a region representing a bone of the biological object (i.e. for automatically identifying bone-regions). Said automatic identification might be realized by running, on the images of the initial set, an algorithm that has been trained to identify a bone region or part in an image received as input. Then, for each image identified as comprising a region representing a bone, the visibility of at least part of said region or of all said region is increased by altering the contrast of said region, in order to render the bone more visible with respect to non-bone tissue. Preferentially, the contrast alteration is performed only for the region of the image comprising a representation of the bone of the biological object, the contrast of any non-bone region remaining preferentially unchanged.

For altering said contrast of the considered MRI image, the system according to the invention, for instance its processing unit, might be configured for applying to the considered MRI image a bone segmentation technique that is configured for separating (or isolating) from the rest of considered MRI image said region of said MRI image representing said bone of the biological object, enabling to partition (or segment) therefore the considered MRI image into two distinct regions, namely said bone region representing the bone of the biological object, and said non-bone region that represents a region of the biological object that is free of a bone. The processing unit might be configured for altering then the contrast of the isolated bone region (and preferentially only of the bone region, i.e. the contrast of the non-bone region remains free of any alteration) to make bone, notably cortical bone, more visible (e.g. brighter) than said biological object tissue. By more visible, it is understood that the contrast intensity difference between the bone region and the non-bone region is increased after applying said contrast alteration. To perform said bone segmentation technique, the system according to the invention, notably its processing unit, may use an analytical model or a deep learning model (e.g. U-Net) or any known in the art segmentation technique configured for or capable of segmenting an image into a bone region and a non-bone region.

In particular, for making bone, notably cortical bone, more visible (e.g. brighter) than said biological object tissue, the method comprises using a function configured for changing a pixel (or voxel) intensity value of each pixel (or voxel) of said isolated bone region, notably so that a difference in intensity values between a voxel belonging to the bone region and a voxel belonging to a non-bone region be increased. For instance, to amplify bone contrast in said isolated bone region, the system, notably its processing unit, may apply to said isolated bone region the following function: $vct \left[x\right] = 2 ⋅ \left(1-vct{\left[x\right]}^{1 / 2}\right) .$ wherein *vct*[***x***] is the intensity value of the voxel located at position **x** within the isolated bone region. Thanks to said function, the intensity values within said isolated bone region might be changed so that voxels with low intensity values have their respective intensity value increased, and vice-versa for voxels with high intensity values. Of course, other functions for modifying the intensity value of voxels within the isolated bone region might be used, the goal being to make any bone region more visible than non-bone region(s).

Alternatively, for rendering said region representing a bone (or part of a bone) of the biological object more visible (e.g. brighter) than non-bone region(s) in said one or several images, the system according to the invention, notably its processing unit, might be configured for automatically creating, for each of said one or several images, a corresponding Computer-Tomography-like image, hereafter CT-like image. In this case, the input set of images comprises then the CT-like images obtained from the one or several images of the initial set. Known in the art techniques might be used for creating a CT-like image from an MRI image. Advantageously, the CT-like image increases the visibility of bones with respect to the "original" MRI image from which it has been generated or created. Alternatively, a 3D CT-like image of the biological object might be directly acquired by the system according to the invention.

According to the present invention, the processed one or several images comprised in the input set are used for generating or creating the IBV 3D image. Said IBV 3D image is a 3D image of the biological object, wherein the visibility of bone(s) in said 3D image has been increased with respect to non-bone region/tissue for enabling said creation of the MRXR image when using it as input to the cone-beam projection algorithm. Typically, the input set comprises MRI images which have been modified for increasing the contrast between the bone region and the non-bone region represented in the image. Said MRI images might be for instance 2D CT-like images, or 2D contrast altered images, or even a 3D contrast altered image. In the case the input set comprises 2D images, then said images (e.g. 2D contrast altered or 2D CT-like images) are used for reconstructing a 3D view of the biological object, that is said IBV 3D image. Known in the art reconstruction techniques might be used for generating the IBV 3D image from the 2D images comprised in the input set. Alternatively, said IBV 3D image might be the 3D contrast altered image (in this case, no reconstruction is needed, given that the 3D contrast altered image is already a three-dimensional representation of the biological object) or a 3D CT-like image of the biological object.

The obtained IBV 3D image can then be used as input to the cone-beam projection algorithm. Indeed, the latter is configured for receiving as input said IBV 3D image (e.g. a contrast-altered 3D image), a source position (the source being typically represented by a point) with respect to the IBV 3D image, and a distance of a 2D projection plane with respect to the source position (or the IBV 3D image position), wherein the MRXR image is the image obtained by projecting the IBV 3D image onto the 2D projection plane when considering rays cast from the source position. Preferentially, each cast ray is associated to a path that is a straight line originating from the source position, passing through the IBV 3D image (and thus intersecting with one or several voxels of the IBV 3D image), and ending onto the 2D projection plane, wherein a pixel is associated to the intersection of the cast ray with the 2D projection plane, wherein an intensity of said pixel is determined in function of a weighted contribution of each voxel of the IBV 3D image that is crossed by the cast ray, and wherein all such pixels arranged on the 2D projection plane form the MRXR image. Preferentially, the weighted contribution is a function of an intensity value of the voxel of the IBV 3D image crossed by the cast ray. Preferentially, the distance between said source position and the IBV 3D image, and respectively said distance between the projection plane and the source position are configured for mimicking real distances separating an X-Ray projection source from an object to be imaged, and respectively said X-Ray projection source from the X-Ray detector plane, so that the rendered MRXR image looks like a real X-Ray image.

### Brief Description of the Drawing

Further aspects of the present invention will be better understood through the following drawings, wherein like numbers designate like objects:
- Fig. 1: schematic illustration of a preferred embodiment of a system according to the invention;
- Fig. 2: flowchart of a preferred embodiment of a method according to the invention;
- Fig. 3: illustration of an MRI image according to the invention;
- Fig. 4: illustration of a contrast-altered image obtained when processing the image of Fig. 3 according to the invention;
- Fig. 5: schematic illustration of a preferred embodiment of a cone-beam projection according to the invention;
- Fig. 6: example of an MRXR image according to the invention.

### Description of Examples

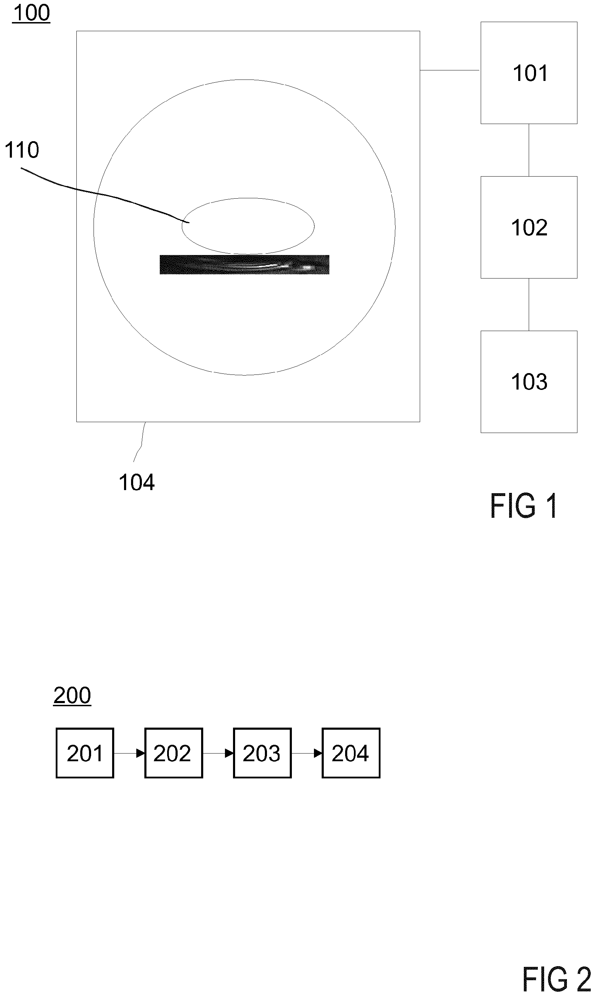

Figure 1 illustrates schematically a preferred embodiment of a system 100 according to the invention. Said system 100 comprises a first interface 101 for acquiring a set of one or several MRI images, a processing unit 102, comprising for instance or one several processor and one or several memories, said processing unit 102 being configured for processing said MRI images, a second interface 103, which might be the same as the first interface 101, for outputting the processed images, notably the MRXR generated through the different steps of the method according to the invention. Said system 100 might be part of an MR apparatus 104, or might be connected to an MR apparatus 104 for acquiring said MR images, said MR apparatus 104 comprising typically a scanner for acquiring MR images of a biological object 110.

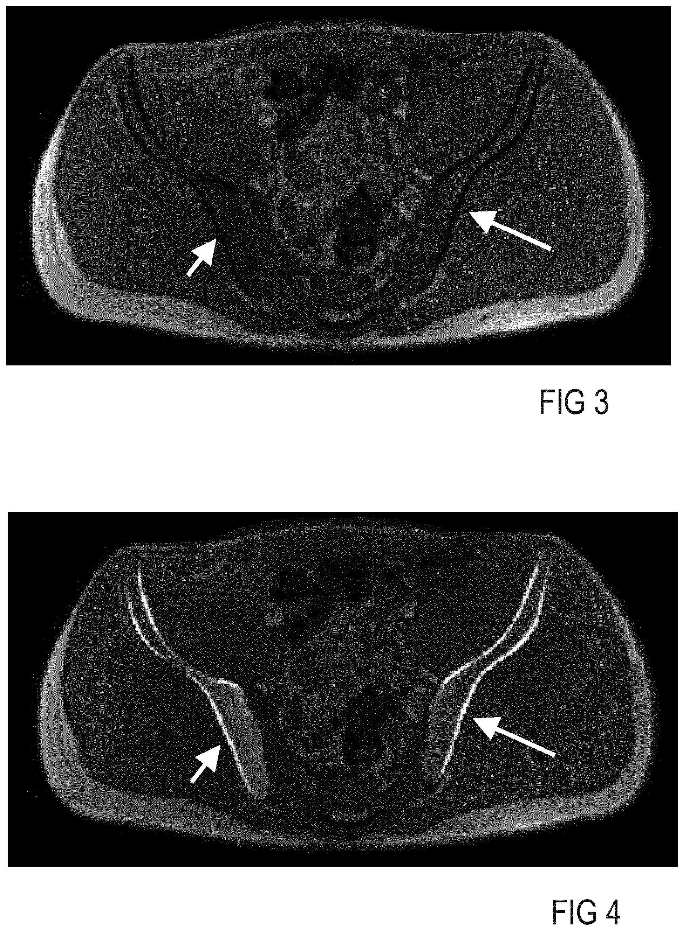

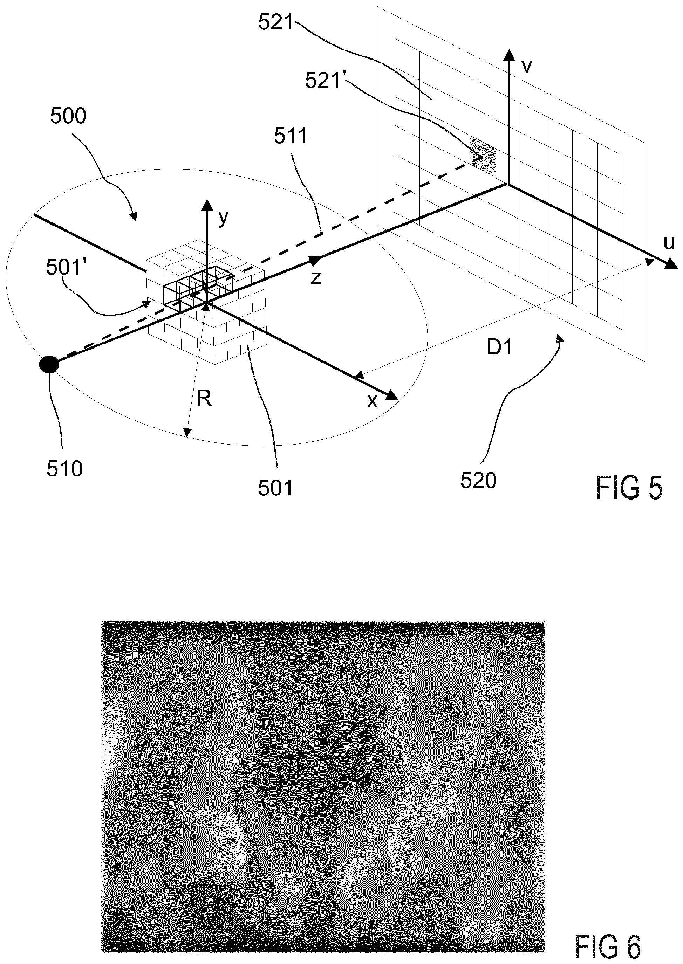

Figure 2 illustrates schematically a preferred embodiment of a method 200 for generating MR virtual X-Ray images according to the invention. The different steps of the method 200 will be described in connection with the Figures 3 to 6, which illustrate respectively an MRI image representing an axial view of a pelvis (Fig. 3), a contrast-altered image of said MRI image representing the axial view of the pelvis shown in Fig. 3 (Fig. 4), a cone-beam projection according to the invention (Fig. 5), and an MRXR image according to the invention (Fig. 6).

At step 201, the system 100 according to the invention acquires or receives, notably via the first interface 101, an initial set of MRI images comprising one or several images of the biological object 110. Said initial set may for instance comprise a single 3D MRI image of the biological object 110, said single 3D MRI image comprising voxels distributed in the three dimensions of space, each voxel being associated to a specific location within the biological object, wherein the intensity (or intensity value) of each voxel is a function of the biological object tissue properties at said specific location associated to the concerned voxel. Alternatively, said initial set may comprise several MRI images, each typically representing a slice of the biological object 110, each image comprising voxels distributed in the two dimensions of space, each voxel being here also a function of the biological object tissue properties at the specific location associated to the concerned voxel. According to the present invention, the images comprised within the initial set shall enable, e.g. using a 3D image reconstruction technique from 2D images, a representation of the (whole) biological object 110 in the three dimensions of space. Typically, if the initial set does not comprise already a 3D MRI image of the biological object 110, then it shall comprise a sufficient number of 2D MRI images for reconstructing a 3D view or representation of the biological object 110, wherein in said 3D representation, the biological object is represented by voxels distributed in the three dimensions of space. The aim of the present invention is to generate an MRXR image of the biological object 110, which means that the visibility of bones in MRI images of said biological object 110 needs to be increased with respect to other tissue (non-bone tissue) of the biological object 110. Typically, it is considered that the 3D representation of the biological object 110 comprises a part (e.g. volume within the 3D representation) representing a bone of the biological object 110, called hereafter the bone part, and another part representing a biological object tissue softer than said bone or free of bone, called hereafter the non-bone part. The same applies mutatis mutandis to the one or several images of the initial set, which may comprise a region representing a bone or part of a bone, i.e. the so-called bone region, and other regions or another region that represents tissue free of any bone, i.e. the so-called non-bone region. Figure 3 typically illustrates a 2D MRI image comprised within an initial set according to the invention. Cortical bone regions (see the arrow) appear dark and have a low visibility.

At step 202, the system 100, notably its processing unit 102, processes said one or several MRI images of the initial set for increasing, for each of said one or several MRI images, a visibility of each (cortical) bone region with respect to a visibility of any non (cortical)-bone region. For instance, the system 100, e.g. said processing unit 102, might be configured for creating, from said one or several MRI images of the initial set, 2D CT-like images enabling a 3D representation of the biological object, or a 3D CT-like image of the biological object 110, or 2D contrast-altered images that enable a 3D representation of the biological object, or a 3D contrast-altered image of the biological object. In particular, known in the art algorithms might be used for creating 2D or 3D CT-like images from an initial set of MRI images. For creating a 2D contrast-altered image from a 2D MRI image of the initial set, or a 3D contrast-altered image from a 3D MRI image of the initial set, the present invention proposes notably to automatically identify, within the image of the initial set, the bone region, i.e. the region within the image that represents a bone or part of a bone of the biological object 110. For this purpose, known in the art segmentation techniques might be used, notably based on a machine learning algorithm that has been trained for identifying any bone region in an image received as input and for outputting a segmented image comprising two regions, the bone region and the non-bone region. Then, the present invention proposes notably to alter the contrast of the bone region (notably only of the bone region of the image, the contrast of the non-bone region remaining for instance unaltered) in order to render (cortical) bone more visible than tissue that is softer than said (cortical bone). For this purpose, and as explained earlier, a function or algorithm might be applied to said bone region, wherein said function or algorithm is configured for changing the intensity value of each voxel of said (cortical) bone region so that its visibility be increased with respect to intensity value of voxels that do not belong to said (cortical) bone region, increasing therefore the contrast between bone region(s) and non-bone region(s). A result is shown in Figure 4, wherein cortical bone of Figure 3 has been made more visible by increasing the intensity value of each voxel belonging to the (cortical) bone region (see the arrows). Said algorithm or function may simply receive as input, for each voxel of the bone region, a voxel intensity value, and return as output, a new value calculated from the input voxel intensity value, wherein said new value is configured for increasing the contrast with respect to voxel intensity values that do not belong to the bone region.

The processing unit 102 is then configured for populating or creating an input set of images with the processed one or several MRI images. Therefore, said input set of images may comprise for instance the 2D contrast-altered images that have been obtained by altering the contrast between the bone region and non-bone regions in the images of the initial set, or the 3D contrast-altered image, or 2D CT-like images obtained from the images of the initial set, or a 3D CT-like image of the biological object 110.

At step 203, the system 100, notably its processing unit 102, is configured for feeding a cone-beam projection algorithm with an IBV 3D image obtained from the processed image(s) comprised in the input set. The IBV 3D image is a 3D representation of the biological object obtained from the images of the initial set after their processing for improving the visibility of bone region(s) with respect to non-bone region(s). It might be obtained for instance by reconstructing a 3D view of the biological object from the 2D images of the input set (said 2D images being for instance contrast-altered 2D images or 2D CT-like images), or it might be the 3D contrast-altered image of the input set, or the 3D CT-like image of said input set. The IBV 3D image is thus in other words a 3D view of the biological object 110 based on or reconstructed from the one or several MRI images of the initial set after the latter undergo said processing for improving or increasing the visibility of a bone region with respect to a non-bone region. The cone-beam projection algorithm is schematically illustrated in Figure 5. It receives as input the IBV 3D image 500, a position of a source 510 of RX with respect to the position of the IBV 3D image 500, and a distance D1 of a 2D projection plane 520 with respect to the position of the source 510 (or of the IBV 3D image). The distance D1 might be measured from the center of the projection plane 520 to the center of the source 510. The source 510 is an X-ray source, which can be schematically represented as a point from which X-ray are emitted. The IBV image 500 is represented as a set of voxels 501 distributed in the three dimensions of space. In Figure 5, each cube of the IBV 3D image 500 schematically represents such a voxel 501. Preferentially, a coordinate system (x,y,z) might be defined with respect to the IBV 3D image for characterizing the position of each voxel of the IBV 3D image in said three dimensions of the space. For instance, the origin of the coordinate system might be located at the centroid of the IBV 3D image, the source being at a distance R from said origin, within the xz plane, wherein the source 510, the center of the projection plane 520, and said origin are preferentially aligned along the z-axis. The projection plane 520 is typically represented as a plane parallel to the xy plane and comprising pixels (i.e. said plane being made of pixels) whose intensity will vary in function of the voxels 501 that are crossed by an X-ray originating from the source 510. Another coordinate system (u,v) might be used for characterizing the position of each pixel of the projection plane. Of course, other ways of representing the system formed by the source 510, the IBV 3D image, and the projection plane 520 might be used by the skilled person.

The MRXR image according to the invention is then the image obtained by projecting the IBV 3D image 500 onto the 2D projection plane 520 when considering rays 511 cast from the source position and passing through the IBV 3D image. According to the present invention, each cast ray 511 is associated to a path that is a straight line connecting the position of the source 510 to a position onto the 2D projection plane 520 and which passes through one or several voxels 501 of the IBV 3D image. The intersection of the cast ray 511 with the 2D projection plane 520 is associated to one of the pixels 521 of the projection plane, and the intensity of said associated pixel depends on a weighted contribution of each voxel of the IBV 3D image that is crossed by the cast ray. For instance, the intensity value of the pixel 521' will depend on the intensity value of each of the 4 voxels 501' (represented in bold) that is crossed by the ray 511. In other words, said weighted contribution is preferentially a function of an intensity value of the voxel crossed by the cast ray, so that a resulting intensity of a pixel of the projection plane 520 will depend on the number of voxels crossed by a ray and their respective intensity: for instance, the intensity value of the pixel might be equal to a normalized sum over the intensity value of the crossed voxels. In particular, the cone-beam projection algorithm according to the invention is configured for using distance parameters that are based on real physical parameters used for performing real X-ray imaging in order to better mimic reality. For instance, the distance R between the position of the source 510 and the position of the IBV 3D image (e.g. its centroid) as well as the distance D1 between the projection plane 520 and the position of the IBV 3D image (e.g. its centroid) that are used by the cone-beam projection algorithm for calculating the intensity value of each pixel of the projection plane 520 are based on real distances separating an X-Ray projection source, an object to be imaged, and an X-Ray detector plane in order to simulate a true or real X-ray imaging, i.e. so that the MRXR image exhibits geometrical features mimicking true X-ray imaging.

At the end, at step 204, the system 100 according to the invention, e.g. its processing unit 102, is configured for outputting the MRXR image, for instance via the second interface 103, wherein the MRXR might be displayed on a screen. The MRXR corresponds to the image formed on the projection plane 520. It is thus a 2D image comprising pixels whose variation of the intensity has been calculated from the intensity value of each of the voxels of the IBV 3D image that has been crossed by a ray connecting the position of the source 510 to the position of the pixel on the projection plane 520. An example of such an MRXR image obtained according to the presently described method is illustrated in Fig. 6.

To conclude, the present invention proposes a method and a system for creating a virtual X-ray image, i.e. the so-called MRXR image, from MRI image(s), wherein said MRXR is the projection, onto a 2D projection plane, of an IBV 3D image that has been created by increasing the visibility of a bone region in said MRI image(s) with respect to a visibility of a non-bone region in said MRI image(s). This is notably realized by increasing the visibility difference between bone region and non-bone region, notably by changing or tuning the contrast intensity of the pixels/voxels of the bone region with respect to contrast intensity of pixels/voxels of non-bone region to render the bone region, notably cortical bone, more visible. This enables a generation of radiation-free virtual radiographs based on MR data which exhibit the same geometrical distortions as true radiographs. Thanks to the MRXR image, diagnostic criteria such as the alpha angle to assess impingement can be evaluated on virtual radiographs, i.e. on MRXR images, instead of true X-Rays, and therefore ionizing radiation can be omitted. Additionally, complementary information can be obtained from a single machine, i.e. the MRI apparatus, saving time and material.

## Claims

1. Method (200) for generating an MR-based-virtual X-Ray, hereafter MRXR, image, said method comprising:
- acquiring (201) an initial set of one or several MRI images, wherein the MRI images comprised within said initial set are configured for enabling one or several three-dimensional - hereafter 3D - representations of a biological object (110);
- processing (202) said one or several MRI images for increasing a visibility of each image region, if any, that represents a bone of the biological object - called hereafter "bone-region" - with respect to a visibility of another region that represents tissue of the biological object that is free of bone - called hereafter "non-bone region", and creating an image input set comprising the processed one or several images;
- feeding (203) an improved bone visibility - hereafter IBV - 3D image (500) into a cone-beam projection algorithm configured for performing an X-Ray-like projection, wherein the IBV 3D image (500) is obtained from the processed one or several images of said input set, and wherein the cone-beam projection algorithm is configured for outputting said MRXR image;
- providing (204) the outputted MRXR image via an interface.

2. Method (200) according to claim 1, wherein said initial set comprises several two-dimensional - hereafter 2D - MRI images that enable said 3D representation of the biological object (110) .

3. Method (200) according to claim 2, wherein the IBV 3D image is reconstructed from the images of the input set.

4. Method (200) according to claim 1, wherein said initial set comprises one MRI image that is a 3D MRI image representing said biological object (110).

5. Method (200) according to one of the claims 1 to 4, wherein processing (202) said one or several MRI images comprises, determining, for each of the MRI images of the initial set, if said MRI image comprises a bone region, and, in the affirmative, replacing said MRI image by a contrast-altered image, wherein the contrast-altered image is obtained by altering a contrast of the considered MRI image for increasing said visibility of said bone region with respect to the visibility of any non-bone region.

6. Method (200) according to claim 5, comprising automatically identifying, within the initial set of one or several MRI images, MRI images that comprise a bone region.

7. Method (200) according to one of the claims 5 or 6, wherein altering said contrast of the considered MRI image comprises applying to the considered MRI image a bone segmentation technique configured for separating or isolating the bone region from the rest of considered MRI image, enabling to partition or segment therefore the considered MRI image into two distinct regions, namely said bone region and said non-bone region, and altering the contrast of the isolated bone region to make bone more visible than biological object tissue of the non-bone region.

8. Method (200) according to claim 7, wherein said bone segmentation technique uses an analytical model or a deep learning model for segmenting an image into a bone region and a non-bone region.

9. Method (200) according to claim 7 or 8, wherein, for making bone more visible than said biological object tissue, the method comprises using a function configured for changing a voxel intensity value of each voxel of said isolated bone region so that bone contrast in said isolated bone region be amplified.

10. Method (200) according to one of the claims 1 to 4, wherein processing (202) said one or several MRI images comprises automatically creating, for each of said one or several MRI images a corresponding Computer-Tomography-like image, hereafter CT-like image, the input set of images comprising thus the CT-like images obtained from the one or several MRI images of the initial set.

11. Method (200) according to one of the claims 1 to 10, wherein the cone-beam projection algorithm is configured for receiving as input said IBV 3D image (500), a source position with respect to the position of the IBV 3D image (500), and a distance of a 2D projection plane (520) with respect to the position of the source (510) or of the IBV 3D image (500), wherein the MRXR image is the image obtained by projecting the IBV 3D image (500) onto the 2D projection plane (520) when considering rays (511) cast from the source position.

12. Method (200) according to claim 11, wherein each cast ray (511) is associated to a path that is a straight line originating from the source position, passing through the IBV 3D image (500), and ending onto the 2D projection plane (520), wherein a pixel (521, 521') of the 2D projection plane (520) is associated to the intersection of the cast ray with the 2D projection plane (520), wherein an intensity of said pixel (521, 521') is determined in function of a weighted contribution of each voxel (501, 501') of the IBV 3D image (500) that is crossed by the cast ray (511), and wherein all such pixels (521, 521') arranged on the 2D projection plane (502) form the MRXR image.

13. Method (200) according to claim 12, wherein said weighted contribution is a function of an intensity value of the voxel (501, 501') crossed by the cast ray (511).

14. Method (200) according to one of the claims 11 to 13, wherein a distance (R) between said source position and the IBV 3D image (500), and respectively said distance between the projection plane and the source position are configured for mimicking real distances separating an X-Ray projection source from an object to be imaged, and respectively said X-Ray projection source from an X-Ray detector plane.

15. System (100) for generating an MR-based virtual X-Ray, hereafter MRXR, image, the system comprising:
- a first interface (101) for acquiring an initial set of one or several MRI images, wherein the MRI images comprised within said initial set are configured for enabling a 3D representation of a biological object (110);
- a processing unit (102) comprising a processor and a memory, said processing unit (102) being configured for processing said one or several MRI images;
wherein said processing unit (102) is configured for carrying out the steps of the method (200) according to one of the claims 1 to 14 .
